# EUROPEAN PATENT APPLICATION

(11) **EP 4 454 609 A1**
(43) Date of publication of application: **30.10.2024**
(21) Application number: 24166742.7
(22) Date of filing: 27.03.2024
(51) Int. Cl.: A61F 2/24, A61M 25/06, A61M 39/06

(54) **PEELABLE ADVANCER FOR DELIVERY SYSTEM**

(30) Priority: 26.04.2023 US 202363498491 P; 21.02.2024 US 202418582976
(71) Applicant: Medtronic, Inc., Minneapolis, MN 55432 (US)
(72) Inventor: Anderson, Marc A., Minneapolis, 55432 (US); Clarke, Luke A., Minneapolis, 55432 (US); McGuinn, Alan Thomas, Minneapolis, 55432 (US); Norgrove, Matthew P., Minneapolis, 55432 (US); Farrell, Timothy Desmond, Minneapolis, 55432 (US); Mullen, Conleth A., Minneapolis, 55432 (US); Walsh, Cian, Minneapolis, 55432 (US); Casley, Mark, Minneapolis, 55432 (US)
(74) Representative: Zimmermann & Partner Patentanwälte mbB

(57) **Abstract**

An advancer (100) includes an advancer hub (130) and an advancer shaft (110) extending distally from the advancer hub. The advancer shaft includes a proximal end (114), a distal end (116), and a lumen (112) extending from the proximal end to the distal end. The advancer shaft is configured to be disposed around an outer shaft of a delivery catheter (200), and includes a split line (118) configured to enable splitting the shaft to enable removal of the shaft from the outer shaft. The advancer hub configured to be mounted on the outer shaft of the delivery catheter, and includes a parting line (142) to enable splitting of the advancer hub.

## Description

### CROSS-REFERENCE TO RELATED APPLICATIONS

This application claims the benefit of U.S. Provisional Patent Application Serial No. 63/498,491, filed April 26, 2023, the entire content of which is incorporated herein by reference.

### FIELD OF THE INVENTION

Embodiments hereof relate to delivery systems and more particularly to peelable advancers for use with delivery systems.

### BACKGROUND OF THE INVENTION

A variety of delivery systems have been used for delivering medical devices such as transcatheter heart valve prostheses. Delivery systems are generally tracked through the vasculature to locate the medical device at a target site. Such delivery systems include catheters that must have flexibility to navigate the twists and turns of the vasculature and sufficient stiffness to be pushed through the vasculature alone or over a guidewire or through a lumen. Such catheters also generally need sufficient stiffness to be pushed through an introducer which is used to gain access to the vasculature. Such introducers are generally located at relatively smaller vessels such that the stiffness needed for catheters is generally greatest at the location of access to the vasculature. Thus, when the distal portion of a catheter is introduced into the vasculature through an introducer, sufficient stiffness is needed to push through the introducer and the access vessel. However, as noted above, flexibility of the distal portion of the catheter is also required to navigate the twists and turns of the vasculature. Thus, a need in the art still generally exists for improved apparatuses and methods for navigating a catheter through or within a patient's anatomy.

### BRIEF SUMMARY OF THE INVENTION

Embodiments hereof relate to an advancer configured for use with a delivery catheter and a delivery system including an advancer.

In a first example, an advancer comprises as shaft and a hub coupled to a proximal end of the shaft. The shaft further includes a distal end and a lumen extending from the proximal end to the distal end. The shaft is configured to be disposed around an outer shaft of a delivery catheter. The shaft includes a split line configured to enable splitting the shaft to enable removal of the shaft from the outer shaft. The hub is also configured to be mounted on the outer shaft of the delivery catheter. The hub includes a parting line to enable splitting of the hub.

In a second example hereof, the advancer of any of the previous or subsequent examples further comprises a seal disposed within the hub, the seal configured to seal the hub against the outer shaft of the delivery catheter, the seal including a seal parting line to enable splitting of the seal.

In a third example hereof, the advancer of any of the previous or subsequent examples further comprises a cap configured to couple the seal to the hub, wherein the cap comprises two cap bodies configured to separate upon splitting of the hub.

In a fourth example hereof, in the advancer of any of the previous or subsequent examples, the shaft includes two split lines disposed about 180 degrees apart around a circumference of the shaft.

In a fifth example hereof, in the advancer of any of the previous or subsequent examples, the hub includes two parting lines disposed about 180 degrees apart around a circumference of the hub.

In a sixth example hereof, in the advancer of any of the previous or subsequent examples, the seal includes two seal parting lines disposed about 180 degrees apart around a circumference of the shaft.

In a seventh example hereof, in the advancer of any of the previous or subsequent examples, the hub includes a hub body and two arms disposed about 180 degrees apart around a circumference of the hub body and extending radially outward form the hub body, wherein the parting line of the hub extend longitudinally along the hub body.

In an eighth example hereof, in the advancer of any of the previous or subsequent examples, the shaft includes a soft tip.

In a ninth example hereof, a system comprises an introducer, a delivery catheter, and an advancer. The introducer includes an introducer hub and an introducer shaft extending distally from the introducer hub. The delivery catheter includes a handle and an outer shaft extending distally from the handle. The advancer includes an advancer hub and an advancer shaft extending distally therefrom. The advancer is mounted over the delivery catheter such that a distal portion of the outer shaft of the delivery catheter extends through the advancer hub and the advancer shaft. The delivery catheter with the advanced mounted thereon is configured to be advanced through the introducer hub and the introducer shaft such that the advancer provides axial stiffness to the delivery catheter as the delivery catheter is advanced through the introducer hub and the introducer catheter. The advancer shaft and the advancer hub are peelable such that the advancer is configured to be removed from around the delivery catheter and from within the introducer after a distal portion of the delivery catheter is advanced distally past a distal end of the introducer shaft.

In a tenth example hereof, the system of any of the previous or subsequent examples further comprises a valve relief component including a valve relief hub and a valve relief shaft, wherein the valve relief component is configured to be disposed within the introducer hub such as to protect a distal portion of the delivery catheter from a seal of the introducer hub.

In an eleventh example hereof, in the system of any of the previous or subsequent examples, the valve relief hub is configured to mate with the introducer hub, and the valve relief shaft is configured to extend through the introducer hub but not extend through the introducer shaft.

In a twelfth example hereof, in the system of any of the previous or subsequent examples, the advancer hub is configured to be disposed proximal of the valve relief hub and the advancer shaft is configured to extend through the valve relief hub, the valve relief shaft, and the introducer shaft.

In a thirteenth example hereof, in the system of any of the previous or subsequent examples, the delivery catheter includes a heart valve prosthesis mounted on a distal portion thereof, wherein a distal end the advancer shaft terminates proximal of the heart valve prosthesis.

In a fourteenth example hereof, in the system of any of the previous or subsequent examples, the delivery catheter is a balloon catheter and the heart valve prosthesis is mounted on a balloon of the balloon catheter.

In a fifteenth example hereof, a method of delivering a medical device to within a vasculature of a patient comprises: introducing an introducer into a vessel of the vasculature, the introducer including an introducer hub having a proximal portion outside of the vessel of the patient and an introducer shaft disposed within the vessel; advancing a delivery catheter through the introducer hub and the introducer shaft, wherein the delivery catheter includes an advancer mounted over an outer shaft of the delivery catheter, wherein the advancer includes an advancer hub and an advancer shaft extending distally from the advancer hub, wherein as the delivery catheter is advanced through the introducer hub and the introducer shaft the advancer is advanced such that the advancer shaft is extends through the introducer hub and the introducer shaft, and a proximal portion of the advancer hub is disposed proximal of the introducer hub; and peeling the advancer from the delivery catheter to remove the advancer from around the delivery catheter and from within the introducer when a distal portion of the delivery catheter has been advanced distal of a distal end of the introducer shaft.

In a sixteenth example hereof, the method of any of the previous or subsequent examples further comprises inserting a valve relief component into the introducer, wherein the valve relief component includes a valve relief hub and a valve relief shaft extending distally from the valve relief hub, wherein advancing the delivery catheter with the advancer mounted thereon comprises advancing the delivery catheter with the advancer mounted thereon through the valve relief component.

In a seventeenth example hereof, in the method of any of the previous or subsequent examples, the advancer shaft includes a split line and the advancer hub includes a parting line, wherein peeling the advancer comprises pulling the advancer hub apart such that the advancer hub and the advancer shaft split along the parting line and the split line.

In a eighteenth example hereof, in the method of any of the previous or subsequent examples, the advancer hub includes a hub body and two arms, wherein the parting line extends longitudinally along the hub body and the two arms extend radially outward from the hub body, and wherein peeling the advancer comprises pulling the two arms apart from each other to split the parting line and the split line.

In an nineteenth example hereof, in the method of any of the previous or subsequent examples, the advancer further comprises a seal configured to seal the advancer hub against the outer shaft of the delivery catheter, the seal including a seal parting line, wherein peeling the advancer comprises splitting of the seal along the seal parting line.

In a twentieth example hereof, in the method of any of the previous or subsequent examples, the advancer comprises a cap configured to couple the seal to the advancer hub, wherein the cap comprises two cap bodies configured to separate upon splitting of the advancer hub.

### BRIEF DESCRIPTION OF DRAWINGS

The foregoing and other features and advantages of the invention will be apparent from the following description of embodiments hereof as illustrated in the accompanying drawings. The accompanying drawings, which are incorporated herein and form a part of the specification, further serve to explain the principles of the invention and to enable a person skilled in the pertinent art to make and use the invention. The drawings are not to scale.
FIG. 1 is a side view of an advancer according to embodiments hereof.
FIG. 2 is a side view and partial cross-section view of the advancer of FIG. 1.
FIG. 3 is a side view of a shaft of the advancer of FIG. 1 according to embodiments hereof.
FIG. 4 is an end view of the shaft of FIG. 2 according to embodiments hereof.
FIG. 5 is a perspective view of a hub of the advancer of FIG. 1 according to embodiments hereof.
FIG. 6 is a distal end view of the hub of FIG. 4 according to embodiments hereof.
FIG. 7 is a proximal end view of the hub of FIG. 4 according to embodiments hereof.
FIG. 8 is a schematic perspective view of a seal of the advancer of FIG. 1 according to embodiments hereof.
FIG. 9 is a schematic perspective view of a cap boy of the advancer of FIG. 1 according to embodiments hereof.
FIG. 10 side cross-sectional view of the cap body of FIG. according to embodiments hereof.
FIG. 11 is a side view of an example delivery catheter.
FIG. 12 is a side view of the example delivery catheter of FIG. 11 and a cross-sectional view of the advancer of FIG. 1 mounted over the example delivery catheter according to embodiments hereof.
FIG. 13 is a side view of an example valve relief component.
FIG. 14 is a side view of the example valve relief component of FIG. 13 mounted over a delivery catheter.
FIG. 15 is a side view of the example valve relief component of FIG. 13 mounted over a delivery catheter and partially disposed within an example introducer.
FIG. 16 is a cross-sectional view of a portion of the advancer of FIG. 1 mounted over an example delivery catheter and disposed within an valve relief component and an introducer accordingly to embodiments hereof.
FIG. 17 is a partial cross-sectional view and partial side view of the advancer of FIG. 1 mounted over an example delivery catheter and disposed within an valve relief component and an introducer accordingly to embodiments hereof.
FIG. 18 is a side view of the example delivery catheter of FIG. 11 and a cross-sectional view of an advancer according to another embodiment hereof.
FIG. 19 is a side view of the example delivery catheter of FIG. 11 and a cross-sectional view of an advancer according to another embodiment hereof.
FIG. 20 is a side view of the example delivery catheter of FIG. 11 and a cross-sectional view of an advancer according to another embodiment hereof.

### DETAILED DESCRIPTION OF THE INVENTION

Specific embodiments of the present invention are now described with reference to the figures, wherein like reference numbers indicate identical or functionally similar elements. The terms "distal" and "proximal" are used in the following description with respect to a position or direction relative to the treating clinician. "Distal" or "distally" are a position distant from or in a direction away from the clinician. "Proximal" and "proximally" are a position near or in a direction toward the clinician. In addition, "slidably" or "slidable" denotes back and forth movement in a longitudinal direction about a longitudinal axis L_{A} of the delivery device while "rotatably" or "rotatable" denotes movement or rotation about the longitudinal axis L_{A} of the handle.

As used in this specification, the singular forms "a", "an" and "the" specifically also encompass the plural forms of the terms to which they refer, unless the content clearly dictates otherwise. The terms "about" and "approximately" are used herein to mean approximately, in the region of, roughly, or around. When the terms "about" and "approximately" are used in conjunction with a numerical range, it modifies that range by extending the boundaries above and below the numerical values set forth. In general, the term "about" is used herein to modify a numerical value above and below the stated value by a variance of 20%. It should be understood that use of the term "about" also includes the specifically recited number of value.

The following detailed description is merely exemplary in nature and is not intended to limit the invention or the application and uses of the invention. Although the description of the invention is in the context of delivery of a balloon-expandable heart valve prosthesis, the invention may also be used where it is deemed useful in endoscopic procedures, procedures in the coronary vessels, or procedures in the peripheral vessels. Furthermore, there is no intention to be bound by any expressed or implied theory presented in the preceding technical field, background, brief summary or the following detailed description.

Embodiments hereof relate to a peelable advancer for use with a delivery system. The peelable advancer is configured to be mounted on an outer shaft of a delivery catheter during advancement of the delivery catheter through an introducer of a delivery system. Upon reaching the distal end of an introducer sheath of the introducer, the peelable advancer can be peeled off of the outer shaft of the delivery catheter and removed from the introducer such that the delivery catheter can be advanced through the vasculature without the peelable advancer attached thereto.

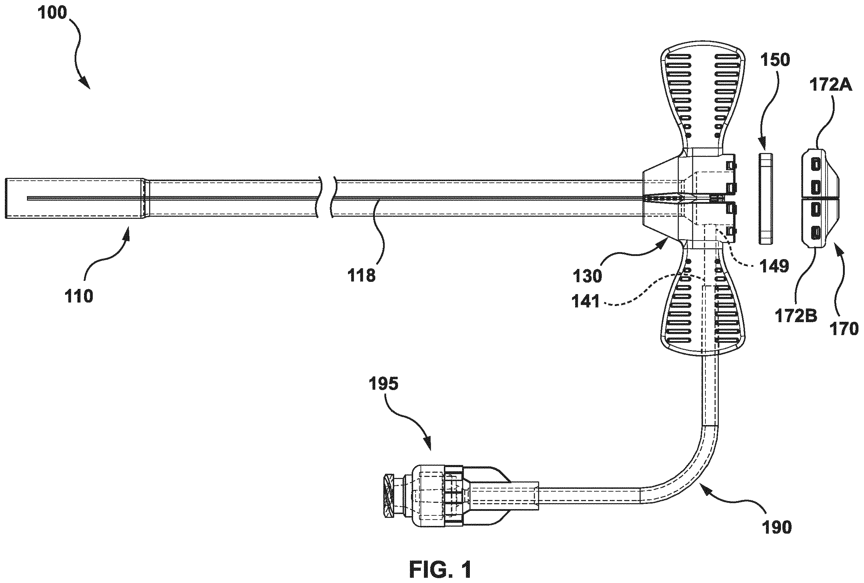

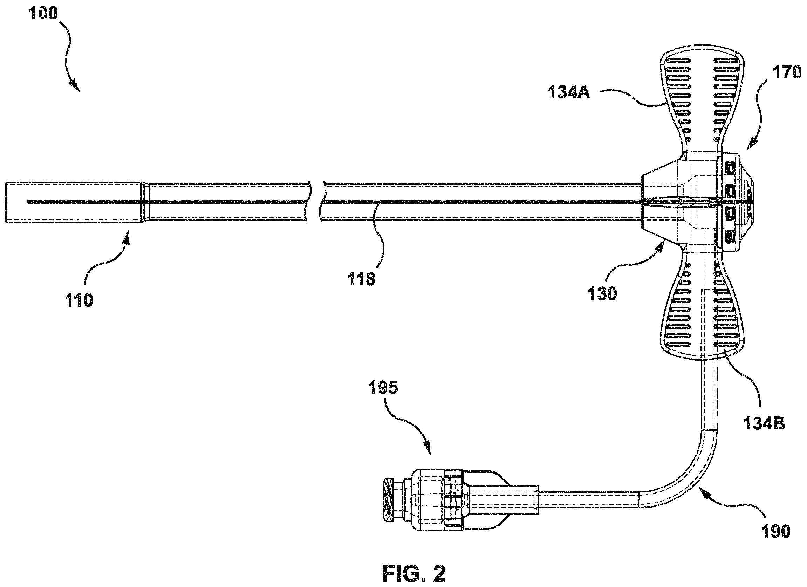

The peelable advancer will now be described in more detail with respect to FIGS. 1-9. FIGS. 1 and 2 show a peelable advancer 100 according to embodiments herein. The peelable advancer 100 includes a shaft 110, a hub 130, a seal 150, a cap 170, a luer tube 190, and a luer valve 195. One skilled in the art will realize that FIGS. 1-9 illustrate one example of a peelable advancer and that existing components illustrated in FIGS. 1-9 may be removed and/or additional components may be added. Components of the peelable advancer 100 will be described below and then the overall peelable advancer 100 will be described referring back to FIGS. 1 and 2.

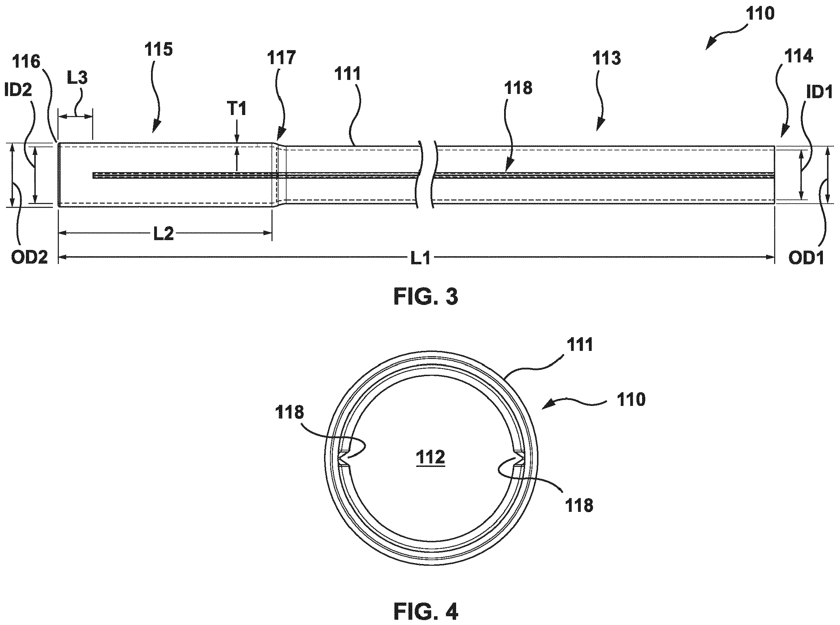

FIGS. 3 and 4 show the shaft 110 of the peelable advancer 100. The shaft 110 is a generally tubular element including having a wall 111 defining a lumen 112 extending from a proximal end 114 to a distal end 116 of the shaft 110. The shaft 110 further includes a proximal portion 113, a distal portion 115, and a transition portion 117 transitioning from the proximal portion 113 to the distal portion 115. The proximal portion 113 has a first outer diameter OD1 and the distal portion 115 has a second outer diameter OD2. In the embodiment shown, the second outer diameter OD2 is larger than the first outer diameter OD1. Similarly, the proximal portion 113 has a first inner diameter ID1 and the distal portion 115 has a second inner diameter ID2, with the second inner diameter ID2 being larger than the first inner diameter ID1. In an embodiment, the first outer diameter OD1 may be about 0.257 inch, the second outer diameter is OD2 may be about 0.286 inch, the first inner diameter ID1 may be about 0.223 inch, the second inner diameter ID2 may be about 0.252 inch, and the wall thickness T1 of the wall 111 may be in the range of about 0.006 inch 0.025 inch. Further, an overall length L1 of the shaft 110 may be in the range of 16 to 20 inches. In an embodiment, the length L1 of the shaft 110 is about 17.9 inches and a length L2 of the distal portion 115 is about 0.965 inch. As would be understood by those skilled in the art, these dimensions may be varied depending on the desired use of the advancer 100, and are not meant to be limiting. For example, and not by way of limitation, the length of the shaft 110 may be longer or shorter if the advancer 100 is used with a longer or shorter introducer, described below. Similarly, the diameters may be larger or smaller depending on dimensions of the outer shaft of the delivery catheter, described below.

The shaft 110 further includes split lines 118 disposed along the length of the wall 111. The split lines 118 enable the shaft 110 to be split and peeled off of the catheter, as described below. In the embodiment shown, there are two split lines 118 disposed 180 degrees apart around the circumference of the shaft 110, as shown in FIG. 4. However, this is not meant to be limiting, and more or fewer split lines may be utilized provided that the split line(s) enable peeling of the shaft 110. The split lines 118 may be formed by reducing the thickness of the wall 111, perforating the wall 111, and/or otherwise creating an area of weakness at the split line to enable a user to separate the wall 111 at the split line. In the embodiment shown the split lines 118 are formed by extrusion die profile. In other words the shaft 110 is extruded, and the die profile makes for a thinner portion of the wall 111 at the split lines 118. In the embodiment shown, the split lines 118 run from the proximal end 114 to proximal of the distal end 116 of the shaft 110. In particular, in the embodiment shown, the split lines 118 stop a length L3 of about 0.16 inch short of the distal end 116 of the shaft 110. The portion of the shaft 110 that does not include the split lines 118 is referred to as the reflow portion, as this reflow portion of the shaft 110 enables force to be transmitted to the distal end of the shaft 110, and provides resistance to the shaft 110 splitting during use instead of when pulled apart by the user.

The wall 111 of the shaft 110 may be formed of various materials suitable for use in the human body, such as but not limited to, polyether block amides (e.g., PEBAX^{®}), polytetrafluoroethylene (PTFE), fluorinated ethylene-propylene copolymer (FEP), perfluoroalkoxy copolymer (PFA), polyurethane, and other similar materials. As shown in FIG. 4, the wall 111 of the shaft 110 may include layers of material. In an embodiment, the wall 111 is formed of PEBAX, Barium sulfate, TINUVIN 622, and TINUVIN 234. In this embodiment, barium sulfate makes the PEBAX brittle enough to be split when peeled, rather than plastically deforming. PEBAX and similar materials need an additive such as barium sulfate to be peelable. However, other materials, such as FEP, are inherently peelable and do not need such an additive. Those skilled in the art will recognize that the materials listed are merely exemplary, are not meant to be limiting, and other suitable materials that are splittable/peelable may also be used.

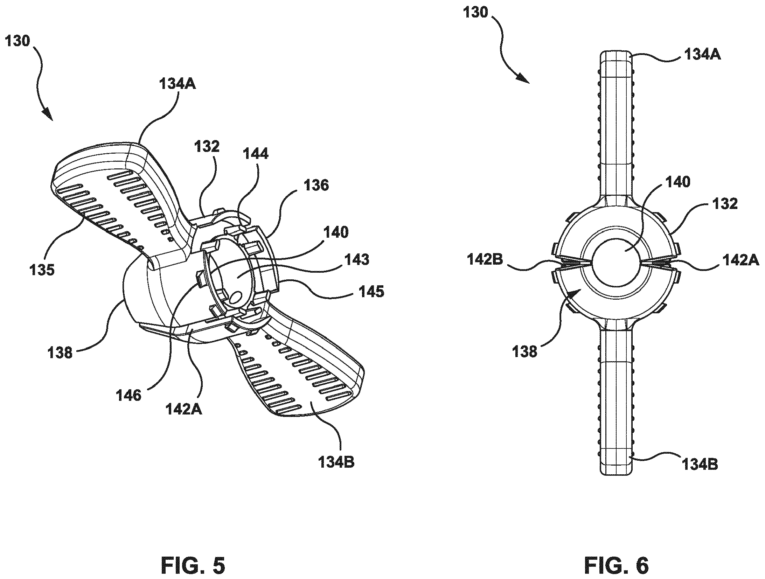

FIGS. 5-7 show the hub 130 of the advancer 100. The hub 130 includes a body 132 and a pair of arms 134A, 134B extending radially outwardly from the body 132. The body 132 include a proximal end 136, a distal end 138, and a lumen 140 extending from the proximal end 136 to the distal end 138. The lumen 140 is configured to receive the shaft 110 of the advancer 100 in a distal portion of the lumen 140, and is configured to receive the outer shaft of the delivery catheter within the entire lumen 140.

As shown in FIGS. 5 and 6, the body 132 includes two parting lines or hub splits 142A, 142B disposed opposite each other around a circumference of the body 132. However, this is not meant to be limiting, and more or fewer parting lines 142 may be included provided that a user is enabled to split the body 132 when the advancer 100 is to be removed from the outer shaft, as explained below. The parting lines 142A, 142B may be formed by reducing the thickness of the wall of the body 132, perforating the wall of the body 132, and/or otherwise creating an area of weakness at the parting line(s) 142 to enable a user to separate the body 132 at the parting lines 142. In the embodiment shown the parting lines 142A, 142B are formed by injection molding the parting lines 142A, 142B to be thinner than the hub body 132.

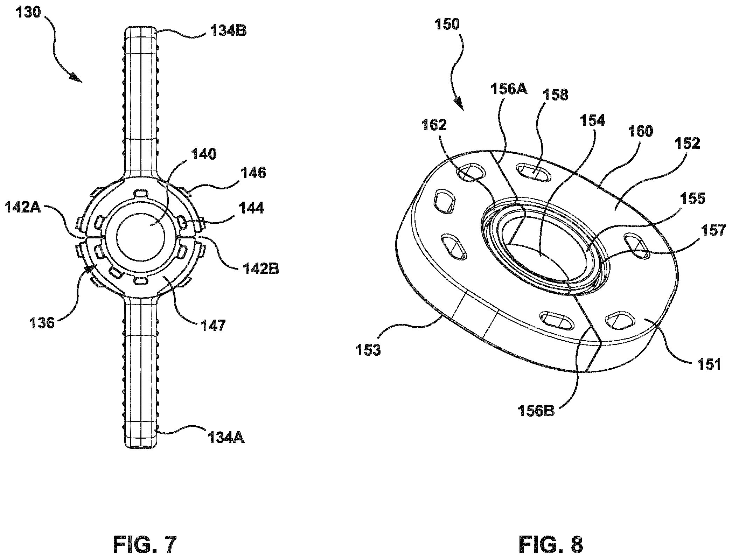

As shown in FIGS. 5 and 7, the proximal portion of the body 132 includes an inner wall 143 defining the lumen 140 and an outer wall 145 defining the outer surface of the body 132. The inner wall 143 and outer wall 145 extend substantially longitudinally. A ledge or shoulder 147 extends between the inner wall 143 and the outer wall 145. The shoulder 147 extends substantially transverse to the central longitudinal axis CLA of the body 132, or substantially radially. Disposed on the outer surface of the inner wall 143 or extending from the shoulder 147 are seal tabs 144. In the embodiment shown, there are seven seal tabs 144, corresponding to seven openings in the seal 150, described below. However, this is not meant to be limiting, and more or fewer seal tabs 144 may be included, or other ways to couple the body 132 to the seal 150 may be utilized. Disposed on the outer surface of the outer wall 145 are cap tabs 146. In the embodiment shown, there are eight cap tabs 146 corresponding to eight snap holes in the cap 160, described below. However, this is not meant to be limiting, and more or fewer cap tabs 146 may be included, or other ways to couple the body 132 to the cap 160 may be utilized

As noted above, the arms 134A, 134B extend radially from the body 132. The arms 134A, 134B are disposed 180 degrees apart around the circumference of the body 132. The arms 134A, 134B are configured for a user to pull the arms 134A, 134B apart from each other to split the body 132 along the parting lines 142A, 142B. Thus, in the embodiment shown, the parting lines 142A, 142B are disposed 90 degrees from each arm 134A, 134B. In other words, the arms 134A, 134B and the parting lines 142A, 142B alternate around the circumference of the body 132. Thus, for example, moving clockwise around the circumference of the body 132 in FIG. 6, the arm 134A is at approximately twelve O'clock, the parting line 142A is at approximately three O'clock, the arm 134B is at approximately six O'clock, and the parting line 142B is at approximately nine O'clock around the circumference of the body 132. Further, the arms 134A, 134B may include ridges 135 or other surface modifications to assist in gripping the arms 134A, 134B to pull the arms 134A, 134B apart to split the body 132 and the shaft 110.

The hub 130 may be made of suitable materials, such as, but not limited to polyethylene block amide, nylon, polycarbonate, and/or other materials known to those skilled in the art.

FIG. 8 shows a perspective view of the seal 150 of the advancer 100. The seal 150 includes a seal body 152 and a central opening 154 such that the seal 150 is generally donut-shaped. The seal body 152 includes a proximal surface 151 and a distal surface 153, with the central opening 154 extending between and through the proximal surface 151 and the distal surface 153. The seal body 152 further includes openings 158 extending between and through the proximal surface 151 and the distal surface 153. The openings 158 are configured such that the seal tabs 144 of the hub 130 can be inserted through the openings 158. Accordingly, in the embodiment shown, there are seven openings 158 corresponding to the seven seal tabs 144. However, this is not meant to be limiting, and more or fewer openings 158 and seal tabs 144 may be utilized, or other ways to couple the seal 150 and the hub 130 may be utilized.

The seal body 152 further includes parting lines 156A, 156B disposed 180 degrees apart from each other around the circumference of the seal body 152. The parting lines 156A, 156B may be a cut through a portion or majority of the seal body 152, or an otherwise weakened area such that the seal body 152 will split along the parting lines 156A, 156B when the arms 134A, 134B of the hub 130 are pulled apart. In the embodiment shown, the parting lines 156A, 156B of the seal 150 are configured to be aligned with the parting lines 142A, 142B of the hub 130 such that when the arms 134A, 134B of the hub 130 are pulled apart from each other, the hub 130 splits at the parting lines 142A, 142B, the seal 150 splits at the parting lines 156A, 156B, and the shaft 110 splits along the split lines 118.

In the embodiment shown, the seal 150 includes an inner wall 155 defining the central opening 154, a mid-wall 157 spaced radially outward from the inner wall 155, and an outer wall 160 defining an outer surface of the seal body 152. A groove 162 is disposed between the inner wall 155 and the mid-wall 157. In the embodiment shown, the groove 162 extend from the proximal surface 151 partially through the seal body 152 such that the groove 162 does not extend to the distal surface 153. In the embodiment shown, the groove 162 is substantially circular and is disposed radially outward of the central opening 154. The groove 162 is configured to receive therein part of the cap 170, described below.

The seal 150 may be made of suitable materials for sealing against an outer shaft of a delivery device to prevent fluid flow therebetween. For example, and not by way of limitation, the seal 150 may be made of silicone rubbers (e.g. Wasker ELASTOSIL LR3003), liquid silicone rubbers, heat cured silicone, and/or other suitable materials known to those skilled in the art.

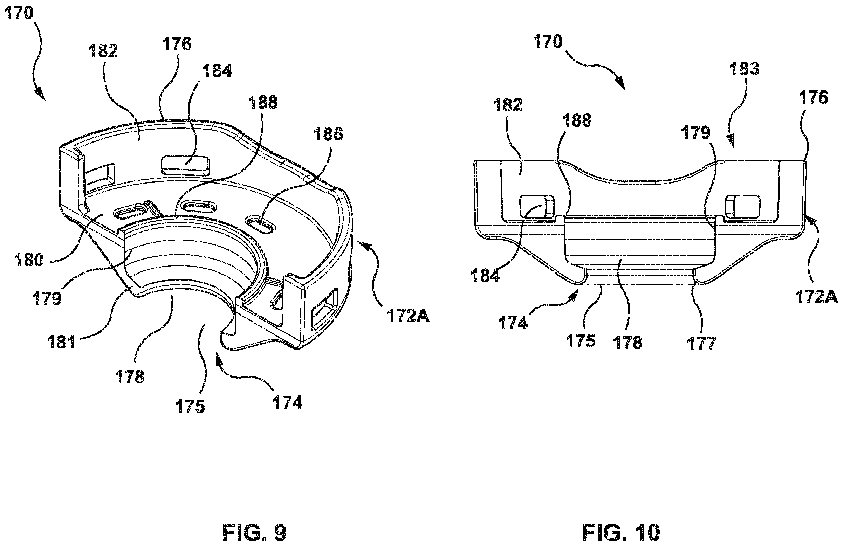

FIGS. 9 and 10 show the cap 170 of the advancer 100. The cap 170 includes two cap bodies 172A, 172B, as shown in FIG. 1. FIGS. 9 and 10 show one of the cap bodies 172A. The other cap body 172B is substantially the same as the cap body 172A, and therefore will not be described separately.

The cap body 172A includes a proximal end 174 and a distal end 176. A lumen or central passage 178 extends from the proximal end 174 to the distal end 176. A proximal opening 175 of the central passage 178 is defined by a flange 174 surrounding the proximal opening 175. The flange 177 extends radially inwardly and proximally from an inner wall 179 of the cap body 172A. A distal surface 181 of the flange 177 is a seat or shoulder that widens the central passage from a first diameter at the proximal opening 175 to a second, larger diameter defined by the inner wall 179. The cap body 172A further includes an outer wall 182 disposed radially outside of the inner wall 179. A distal end of the outer wall 182 defines a distal opening 183 of the central passage 178. A shoulder 180 extends radially outward between the inner wall 179 and the outer wall 182. The inner wall 179 extends distally beyond the shoulder 180, thereby creating a ridge 188 extending distally from an inner circumference of the shoulder 180. The ridge 188 is configured to mate with the groove 162 of the seal 150 with the advancer 100 assembled.

The outer wall 182 includes snap holes 184 extending radially through the outer wall 182. The snap holes 184 are positioned, sized, and shaped (i.e., configured) to receive the cap tabs 146 of the hub body 132 with the advancer 100 assembled. The shoulder 180 includes recesses 186. The recessed are position, sized, and shaped (i.e., configured) to receive the seal tabs 144 of the hub body 132 with the advancer 100 assembled. In the embodiment shown, the cap bodies 172A, 172B are not attached to each other. Instead, they abut each other when attached to the hub body 132. However, this is not meant to be limiting, and the cap bodies 172A, 172B may be releasably attached to each other such that when the advancer 100 is split or peeled, the cap bodies 172A, 172B separate.

The advancer 100 further includes luer tubing 190 and a luer valve 195. The luer tubing and the luer valve 195 are parts known in the art, and as such, will not be described in detail herein.

Referring back to FIGS. 1 and 2, the assembly of the advancer will now be described. The proximal end 114 of the shaft 110 is inserted into the distal end 138 of the hub body 132 such that the proximal end 114 of the shaft 110 is disposed within a distal portion of the lumen 144 of the hub body 132. That shaft 110 is inserted into the hub body 132 such that the split lines 118 of the shaft 110 are aligned with the parting lines 142A, 142B of the hub body 132. The shaft 110 may be attached to the hub body 132 via adhesive, heat bonding, mechanical attachment (e.g., teeth, barbs, wrap, interference fit) and/or other attachment means.

Still referring to FIGS. 1 and 2, the seal 150 is inserted into the proximal end of the hub body 132 such that the seal tabs 144 of the hub body 132 extend through the openings 158 in the seal body 152. The seal 150 is inserted to the hub body 132 with the parting lines 156A, 156B of the seal 150 aligned with the parting lines 142A, 142B of the hub body 132. The distal surface 153 of the seal 150 abuts against the shoulder 147 of the hub body 132. The inner surface of the inner wall 155 extends radially inward of the inner wall 143 of the hub body 132 such that the seal 150 creates a seal against the outer surface of the outer shaft of the delivery catheter, explained below.

With the seal 150 inserted into the hub body 132, the two cap bodies 172A, 172B are advanced over the hub body 132 such that the cap tabs 146 on the outer surface of the outer wall 145 of the hub body 132 extend radially outward through the snap holes 184 of the cap bodies 172A, 172B in a snap fit connection. The abutting lines of the cap bodies 172A, 172B are aligned with the parting lines 142A, 142B of the hub body 132. Further, the seal tabs 144 of the hub body 132 extend through the openings 158 in the seal body 152 and into the recesses 186 of the cap 170. Thus, the seal 150 is captured between the shoulder 147 of the hub body 132 and the shoulders 182 of the cap bodies 172A, 172B.

A first end of the luer tubing 190 is attached to the hub body 132 via an opening 149 in the hub body 132 (see FIG. 1) by an adhesive, heat bonding, or other attachment means. In the embodiment shown, the luer tubing 190 enters the hub 132 through an opening 141 through one of the arms 134A of the hub 130. However, this is not meant to be limiting, and the luer tubing 190 may be in the fluid communication with lumen 112 of the shaft 110 at other locations. A second end of the luer tubing 190 is attached to the luer valve 195 by an adhesive, heat bonding, or other attachment means. The luer tubing 190 and luer valve 195 enable flushing of air from the lumen 112 of the shaft 110 by injecting saline into the lumen 112 using a syringe, for example.

As briefly explained above, the advancer 100 described herein is configured to be used with a delivery system for delivering a medical device, such as a transcatheter heart valve prosthesis, through the vasculature to a treatment site. In particular, the advancer 100 is used adjacent the access point into the vasculature, for example, a femoral artery. Some parts of an exemplary delivery system will be described herein for understanding of the advancer 100 and its use.

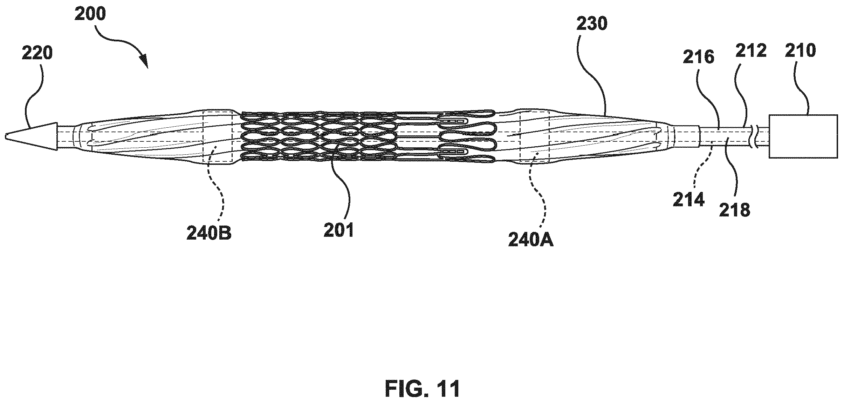

FIG. 11 shows a schematic illustration of a delivery catheter 200 for delivering a heart valve prosthesis 201 to the desired treatment site. The delivery catheter 200 is a balloon catheter and the heart valve prosthesis 201 is balloon expandable such that the delivery catheter can be used to deliver the heart valve prosthesis 201 to the treatment site in a radially compressed or crimped configuration as shown in FIG. 11, and then radially expand the heart valve prosthesis at the treatment site, as known to those skilled in the art. In the example shown, the delivery catheter includes a handle 210 configured to remain outside of the patient's body and used to control the delivery catheter 200, such as by steering, advancing, providing inflation fluid, etc. In the example shown, extending distally from the handle 210 are an inner shaft 214 and an outer shaft 212 surrounding the inner shaft 214. The inner shaft 214 extends to a distal tip 220 at a distal end of the delivery catheter 200. A guidewire lumen 218 extends through the inner shaft 214 and the distal tip 220. The outer shaft 212 surrounds and is spaced from the inner shaft 214 such that an inflation lumen 216 is defined between and outer surface of the inner shaft 214 and an inner surface of the outer shaft 212. The outer shaft 212 stops distally adjacent a proximal end of a balloon 230 such that the inflation lumen 216 opens into an interior of the balloon 230. A proximal end of the balloon 220 is attached to the distal end of the outer shaft 212 and a distal end of the balloon 230 is attached to the distal tip 214 or a distal portion of the inner shaft 214. In the example shown, the delivery catheter 200 includes retention bumpers 240A, 240B proximal and distal of the portion of the balloon 230 where the heart valve prosthesis 201 is crimped. The retention bumpers 240A, 240B may be as described in U.S. Patent Application Publication No. 2022/0054264 A1, which is incorporated by reference herein in its entirety. The delivery catheter 200 is an example only and the advancer 100 described herein and the method described herein may be utilized with other delivery catheters. For example, and not by way of limitation, the advancer 100 and methods described herein may be used with balloon catheters without retention bumpers, with different inflation lumen arrangements, and/or delivery catheters for delivering self-expanding prostheses.

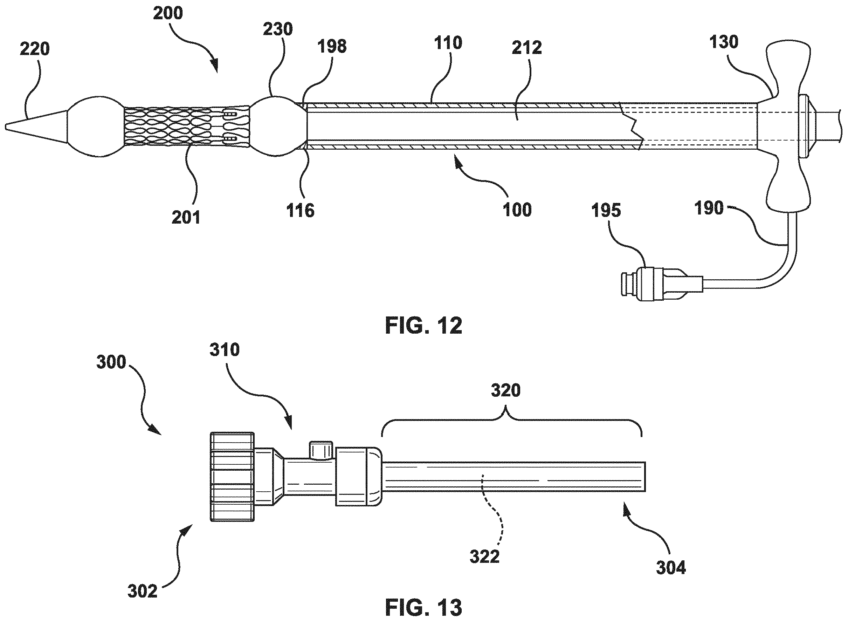

FIG. 12 shows the advancer 100 mounted on the delivery catheter 200. In particular, the advancer 100 is disposed on the outer shaft 212 of the delivery catheter 100, with the distal end 116 of the shaft 110 of the advancer 100 abutting the proximal end of the balloon 230 of the delivery catheter 200. The distal end 116 of the shaft 110 may include a soft tip 198 to abut and/or seal against the balloon 230 to prevent damage to the balloon 230. Thus, the soft tip 198 of the shaft 110 may have a durometer lower than the remainder of the shaft 110. It is noted that the overall length of the advancer 100 (including the shaft 110, the hub 130, and the cap) is significantly less than the length of the delivery catheter 200. Thus, the hub 130 is disposed around the outer shaft 212 of delivery catheter 200 distal of the handle 210 of the delivery catheter 200. For example, and not by way of limitation, in an embodiment, the overall length of the assembled advancer 100 may be about 18.5 inches (about 47 cm) and the length of the outer shaft 212 of the delivery catheter 200 may be about 40 inches to 50 inches (about 100 cm to about 130 cm). Thus, with the distal end of the shaft 110 of the advancer generally aligned with the distal end of the outer shaft 212, the outer shaft 212 of the delivery catheter extends proximal of the advancer 200.

FIGS. 13 shows schematically a valve relief component that may be utilized with the delivery catheter 200 and the advancer 100. FIG. 13 shows a side view of the valve relief component 300. The valve relief component 300 includes a proximal end 302 and a distal end 304. A hub 310 including a hemostasis valve or seal is disposed at the proximal end 302 of the valve relief component 300. The hemostasis valve or seal of the hub 310 may be formed from a flexible material such as silicone and may include a lubricious coating such as parylene or silicone oil. The hemostasis valve or seal of the hub 310 is configured to passively or actively seal against the shaft 110 of the advancer 100 and/or the outer shaft 212 of the delivery catheter 200 when the shaft 110/outer shaft 212 is disposed therethrough, creating hemostasis. Distally extending from the hub 310 is a sheath 320. The sheath 320 is a tubular or cylindrical element defining a single lumen 322 therethrough. The sheath 320 is sized to be used with an introducer sheath with the lumen 322 being sized or configured to slidingly receive the shaft 110/outer shaft 212. The length of the sheath 320 is such that with the hub 310 engaged with a hub of an introducer (described below), the sheath 320 extends through the hub of the introducer. The sheath 320 may be formed of one or more relatively rigid polymeric materials such as but not limited to nylon. Optionally, the sheath 320 or some portion thereof may be formed as a composite having a reinforcement layer incorporated within a polymeric body in order to enhance strength and/or flexibility.

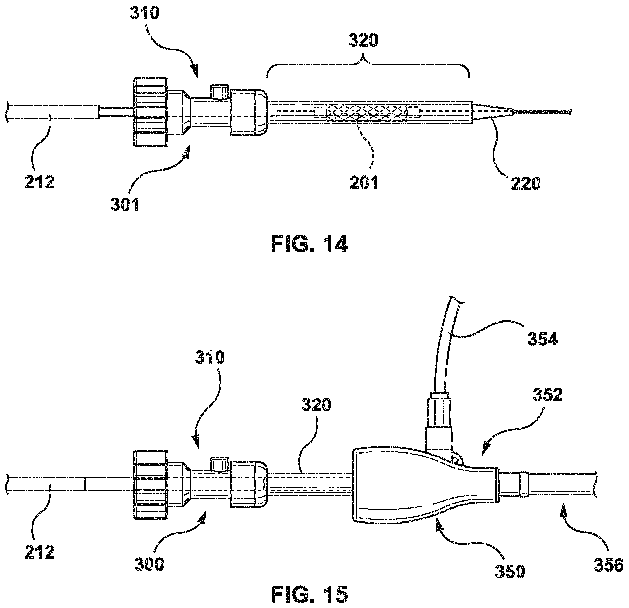

FIG. 14 is a side view of the distal portion of the delivery catheter 200 with the sheath 320 of the valve relief component 300 disposed over the balloon-expandable heart valve prosthesis 201. When positioned over the balloon-expandable heart valve prosthesis 201, the valve relief component 300 reduces or eliminates the external forces that the heart valve prosthesis 201 experiences when loaded through a hemostatic valve of an introducer 350, as shown in FIG. 14. FIG. 15 is a side view of the distal portion of the catheter 300 as the catheter is being inserted through an introducer hub 352 of an introducer 350 with the valve relief component 300 disposed over the heart valve prosthesis 201. The introducer hub 352 of the introducer 350 includes a hemostasis valve therein as well as a flush port 354. The introducer 350 also includes an introducer sheath 356 extending distally from the introducer hub 352, as known to those skilled in the art. The description herein of the valve relief component 300 and the introducer 350 are merely exemplary, and are not meant to be limiting. Further description of an example valve relief component may be found in U.S. Patent No. 11,446,470 to Medtronic, Inc., which is incorporated by reference herein in its entirety.

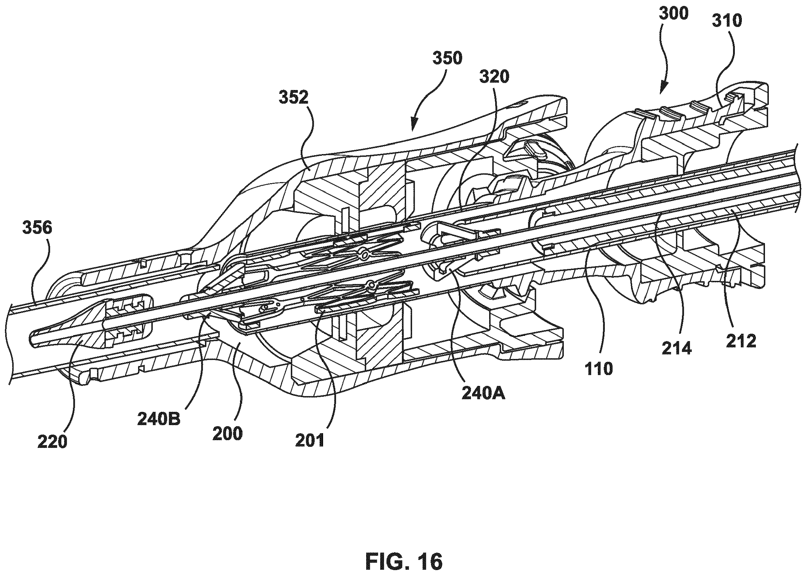

FIG. 16 is a schematic cross-section view of the advancer 100 in use with the delivery catheter 200, the valve relief component 300, and the introducer 350. The balloon 230 of the delivery catheter 200 is excluded for clarity, but as can be seen in FIG. 11, the balloon 230 is attached to the distal end of the outer shaft 212 of the delivery catheter 200, covers the proximal retention bumper 240A, extends under the heart valve prosthesis 201, covers the distal retention bumper 240B, and is attached at its distal end to the distal tip 220 of the catheter 200.

The introducer 350 with the introducer sheath 356 extending distally from the introducer hub 352 is disposed at an access point of the vasculature. For example, and not by way of limitation, the introducer hub 352 is disposed outside of the femoral artery and the introducer sheath 356 is disposed within the femoral artery at the access point and extends within the vasculature therefrom. A can be seen in FIG. 16, the valve relief component 300 is disposed within the introducer hub 352, with the valve relief hub 310 coupled to the introducer hub 352, and the valve relief shaft 320 extending through the seals of the introducer hub 310. As can also be seen in FIG. 16, the valve relief component 300 is disposed over the delivery catheter 200. In particular, as the transcatheter heart valve prosthesis 201 mounted on the balloon of the delivery catheter 200 is advanced through the introducer hub 352, the valve relief component 300 covers the heart valve prosthesis 201 such as to protect the heart valve prosthesis 201 from seals of the introducer hub 320.

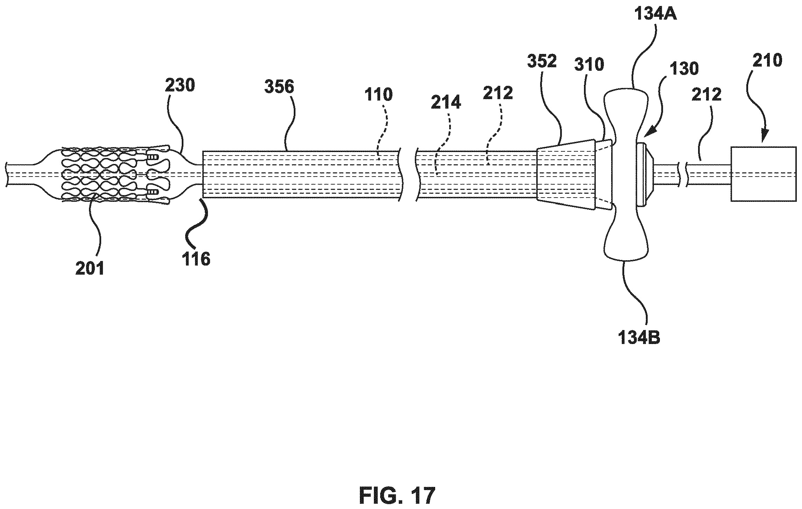

As also shown in FIG. 16, the advancer 100 is disposed over the outer shaft 212 of the delivery catheter 200. In particular, FIG. 16 shows the shaft 110 of the advancer 100 disposed over the outer shaft 212 of the delivery catheter 200, with a distal end of the shaft 110 abutting the balloon (not shown in FIG. 16) of the delivery catheter 201. FIG. 16 shows the delivery catheter 200 and the advancer 100 with the distal portion of the delivery catheter 200 within the introducer hub 352. In such a position, the heart valve prosthesis 201, the distal portion of the shaft 110 of the advancer 100, and the distal portion of the outer shaft 212 of the delivery catheter 201 are disposed within the valve relief component 300, which is in turn disposed within the introducer hub 352. The delivery catheter 200 and the advancer 100 are advanced through the valve relief component 300 and the introducer 350 until the advancer hub 130 of the advancer 100 abuts the valve relief hub 310 of the valve relief component 300, as shown schematically in FIG. 17. Due to the relative lengths of the advancer shaft 110 with respect to the valve relief hub 310, the introducer hub 352, and the introducer sheath 356, the advancer hub 130 abuts the valve relief hub 310 when the distal end 116 of the advancer shaft 110 reaches the distal end of the introducer sheath 356. As explained above, the advancer 100 provides axial strength for the delivery catheter 200 while the delivery catheter 200 is being pushed or advanced through the valve relief component 300, the introducer hub 352 and the introducer sheath 356. However, once the distal portion of the delivery catheter 200 with the heart valve prosthesis 201 is advanced distally past the introducer sheath 356, it is no longer desirable for the advancer shaft 110 to be disposed within the introducer sheath 356, particularly in instances that the introducer sheath 356 is expandable, at least because it is undesirable to maintain the introducer sheath 356 in an expanded configuration for potential trauma to the vessel tissue surrounding the introducer sheath 356.

Therefore, when the distal portion of the delivery catheter 201 with the heart valve prosthesis 201 has been advanced distally past the distal end of the introducer sheath 356, it is desirable to remove the advancer 100 from within the introducer 300. However, there is significant length of the outer shaft 212 of the delivery catheter 200 proximal of the advancer hub 130, and the advancer 100 cannot be removed over the handle 210 of the delivery catheter 200. Therefore, when it is desirable to remove the advancer 100 from the outer shaft 212 of the delivery catheter 200, the arms 134A, 134B of the advancer hub 130 may be pulled proximally and apart. Pulling the arms 134A, 134B of the advancer hub 130 proximally and apart causes the advancer hub 130 to split along the parting lines 142A, 142B, the cap bodies 172A, 172B to separate from each other, the seal 150 to split along the parting lines 156A, 156B, and the advancer shaft 110 to split along the split lines 118, thereby enabling the advancer 100 to be removed from around the outer shaft 212 of the delivery catheter 200, and removed from within the valve relief component 300 and the introducer 350.

It is noted that although the advancer 100 has been described as being used with a system including a delivery catheter 200, a valve relief component 300, and an introducer 350, this is not meant to be limiting, and fewer or more components may be utilized. In particular, for example, and not by way of limitation, the advancer 100 described herein may be used in a system that does not include a valve relief component.

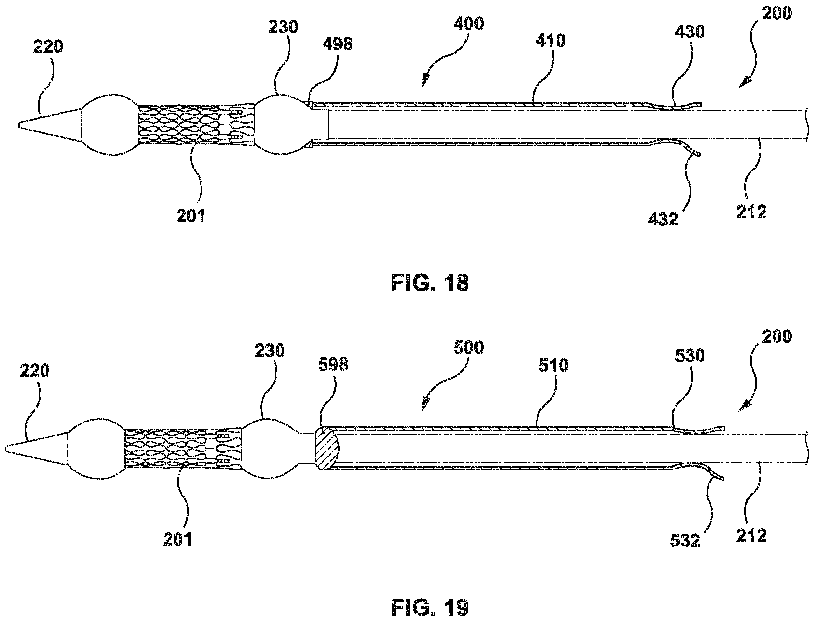

FIG. 18 shows schematically an advancer 400 according to embodiments hereof. The advancer 400 shown in FIG. 18 may include the features of the advancer 100 described above, except where specifically noted. Further, features of the advancer 400 may be incorporated into the advancer 100, as appropriate. The advancer 400 of FIG. 18 is shown on a delivery catheter 200 as described above. Further, although not shown, the advancer 400 may be used with the valve relief component 300 and the introducer 350 shown above in a similar fashion as described above, except where specifically noted.

The advancer 400 includes a shaft 410 similar to the shaft 110. Although not shown in FIG. 18 because FIG. 18 is a schematic partial cross-sectional view, the shaft 410 of the advancer 400 includes split lines such as split lines 118 of the shaft 110 such that the shaft 410 may be split or peeled after being advanced through the introducer 350, as described above. The shaft 410 includes a soft tip 498 as described above to abut and/or seal against the balloon 230 to prevent damage to the balloon 230. Thus, as explained above, the soft tip 498 of the shaft 410 may have a durometer lower than the remainder of the shaft 410.

The advancer 400 shown in FIG. 18 does not include a hub. Instead, a proximal end of the advancer 400 is configured to form a seal 430 against the outer surface of the outer shaft 212 of the delivery catheter 200, as shown in FIG. 18. The seal 430 may be formed via heat shrink, adhesive, or other means know to those skilled in the art. The seal 430 may be formed around the entire circumference of the interface between the advancer shaft 410 and the outer shaft 212 of the delivery catheter. The seal 430 may be formed distal of the proximal edge of the advancer 400, leaving a tab 432 proximal of the seal 430. The tab 432 may extend around an entire circumference of the advancer 400, or may extend around only a portion of the circumference. In an embodiment, the tab 432 may be reinforced and/or may include surface modifications such as ridges to aid in pulling the tab 432.

The advancer 400 is configured to be used in the same manner as the advancer 100, except that when the advancer 400 is removed, the tab 432 is pulled proximally and apart to split or peel the shaft 410 and remove the advancer 400 from around the outer shaft 212 of the delivery catheter 200 and to remove the advancer 400 within the introducer 350 and the valve relief component 300.

FIG. 19 shows an advancer 500 according to embodiments hereof. The advancer 500 shown in FIG. 19 may include the features of the advancer 100 and/or the advancer 400 described above, except where specifically noted. Further, features of the advancer 500 may be incorporated into the advancer 100, as appropriate. The advancer 500 of FIG. 19 is shown on a delivery catheter 200 as described above. Further, although not shown, the advancer 500 may be used with the valve relief component 300 and the introducer 350 shown above in a similar fashion as described above, except where specifically noted.

The advancer 500 includes a shaft 510 similar to the shaft 110. Although not shown in FIG. 19 because FIG. 19 is a schematic partial cross-sectional view, the shaft 510 of the advancer 500 includes split lines such as split lines 118 of the shaft 110 such that the shaft 510 may be split or peeled after being advanced through the introducer 350, as described above. The advancer 500 shown in FIG. 19 is configured to form a seal 530 against the outer surface of the outer shaft 212 of the delivery catheter 200, as described above with respect to the advancer 400. Similarly, the advancer 500 may include a tab 532 proximal of the seal 530 to aid in splitting or peeling the advancer 500, as described above with respect to the advancer 400.

Instead of the soft tip 198/498 at a distal end of the shaft 110/410 of the advancer 100/400 described above, the outer shaft 212 of the delivery catheter 200 includes a soft polymer bumper 598 disposed around the outer shaft 212 adjacent the proximal neck of the balloon 230. The bumper 598 is configured such that a distal end of the advancer shaft 510 abuts against the bumper 598 when the advancer 500 is located over the shaft 212 to provide increased axial stiffness when pushing the delivery catheter 200 through the valve relief component 300 and the introducer 350. It is noted that the bumper 598 may be used with the advancer 100 instead of or in addition to the soft tip 198 described with respect to the advancer 100.

The advancer 500 is configured to be used in the same manner as the advancer 100, except that when the advancer 500 is removed, the tab 532 is pulled proximally and apart to split or peel the shaft 510 and remove the advancer 500 from around the outer shaft 212 of the delivery catheter 200 and to remove the advancer 500 from within the introducer 350 and the valve relief component 300.

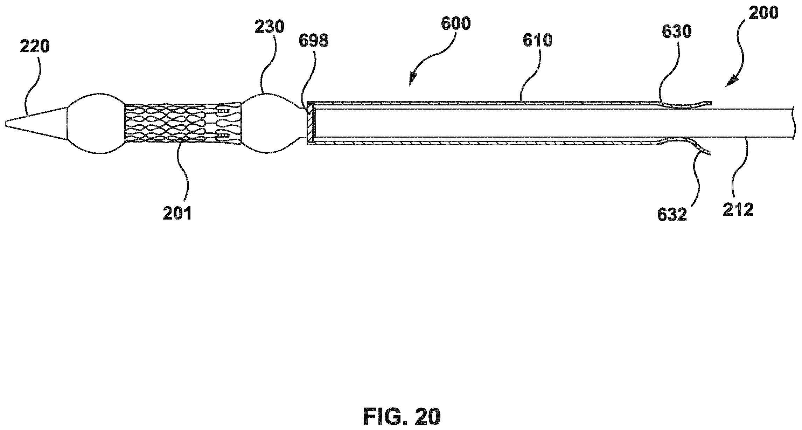

FIG. 20 shows an advancer 600 according to embodiments hereof. The advancer 600 shown in FIG. 20 may include the features of the advancer 100 and/or the advancer 400 described above, except where specifically noted. Further, features of the advancer 600 may be incorporated into the advancer 100, as appropriate. The advancer 600 of FIG. 20 is shown on a delivery catheter 200 as described above. Further, although not shown, the advancer 600 may be used with the valve relief component 300 and the introducer 350 shown above in a similar fashion as described above, except where specifically noted.

The advancer 600 includes a shaft 610 similar to the shaft 110. Although not shown in FIG. 20 because FIG. 20 is a schematic partial cross-sectional view, the shaft 610 of the advancer 600 includes split lines such as split lines 118 of the shaft 110 such that the shaft 610 may be split or peeled after being advanced through the introducer 350, as described above. The advancer 600 shown in FIG. 20 is configured to form a seal 630 against the outer surface of the outer shaft 212 of the delivery catheter 200, as described above with respect to the advancer 400. Similarly, the advancer 600 may include a tab 632 proximal of the seal 630 to aid in splitting or peeling the advancer 600, as described above with respect to the advancer 400.

Instead of the soft tip 198/498 at a distal end of the shaft 110/410 of the advancer 100/400 described above, or the bumper 598, the outer shaft 212 of the delivery catheter 200 includes an O-ring seal 698 disposed around the outer shaft 212 adjacent the proximal neck of the balloon 230. The O-ring seal 698 is configured to seal with a distal end of the advancer shaft 610 when the advancer 600 is located over the shaft 212 to provide increased axial stiffness when pushing the delivery catheter 200 through the valve relief component 300 and the introducer 350. It is noted that the bumper 698 may be used with the advancer 100 instead of or in addition to the soft tip 198 described with respect to the advancer 100.

The advancer 600 is configured to be used in the same manner as the advancer 100, except that when the advancer 600 is removed, the tab 632 is pulled proximally and apart to split or peel the shaft 610 and remove the advancer 600 from around the outer shaft 212 of the delivery catheter 200 and to remove the advancer 600 from within the introducer 350 and the valve relief component 300.

While various embodiments according to the present invention have been described above, it should be understood that they have been presented by way of illustration and example only, and not limitation. It will be apparent to persons skilled in the relevant art that various changes in form and detail can be made therein without departing from the spirit and scope of the invention. Thus, the breadth and scope of the present invention should not be limited by any of the above-described exemplary embodiments, but should be defined only in accordance with the appended claims and their equivalents. It will also be understood that each feature of each embodiment discussed herein, and of each reference cited herein, can be used in combination with the features of any other embodiment. All patents and publications discussed herein are incorporated by reference herein in their entirety.

Further disclosed herein is the subject-matter of the following clauses:
Clause 1. An advancer comprising:
   a shaft including a proximal end, a distal end, and a lumen extending from the proximal end to the distal end, the shaft configured to be disposed around an outer shaft of a delivery catheter, the shaft including a split line configured to enable splitting the shaft to enable removal of the shaft from the outer shaft; and
   a hub coupled to the proximal end of the shaft, the hub configured to be mounted on the outer shaft of the delivery catheter, the hub including a parting line to enable splitting of the hub.
Clause 2. The advancer of clause 1, further comprising a seal disposed within the hub, the seal configured to seal the hub against the outer shaft of the delivery catheter, the seal including a seal parting line to enable splitting of the seal.
Clause 3. The advancer of clause 2, wherein the seal includes two seal parting lines disposed about 180 degrees apart around a circumference of the shaft.
Clause 4. The advancer of clause 2 or 3, further comprising a cap configured to couple the seal to the hub, wherein the cap comprises two cap bodies configured to separate upon splitting of the hub.
Clause 5. The advancer of clause 1 or any preceding clause, wherein the shaft includes two split lines disposed about 180 degrees apart around a circumference of the shaft.
Clause 6. The advancer of clause 1 or any preceding clause, wherein the hub includes two parting lines disposed about 180 degrees apart around a circumference of the hub.
Clause 7. The advancer of clause 1 or any preceding clause, wherein the hub includes a hub body and two arms disposed about 180 degrees apart around a circumference of the hub body and extending radially outward form the hub body, wherein the parting line of the hub extend longitudinally along the hub body.
Clause 8. The advancer of clause 1 or any preceding clause, wherein the shaft includes a soft tip.
Clause 9. A system comprising:
   an introducer, the introducer comprising an introducer hub and an introducer shaft extending distally from the introducer hub;
   a delivery catheter, the delivery catheter including a handle and an outer shaft extending distally from the handle; and
   an advancer, the advancer including an advancer hub and an advancer shaft extending distally therefrom,
   wherein:
   the advancer is mounted over the delivery catheter such that a distal portion of the outer shaft of the delivery catheter extends through the advancer hub and the advancer shaft;
   the delivery catheter with the advanced mounted thereon is configured to be advanced through the introducer hub and the introducer shaft such that the advancer provides axial stiffness to the delivery catheter as the delivery catheter is advanced through the introducer hub and the introducer catheter; and
   the advancer shaft and the advancer hub are peelable such that the advancer is configured to be removed from around the delivery catheter and from within the introducer after a distal portion of the delivery catheter is advanced distally past a distal end of the introducer shaft.
Clause 10. The system of clause 9, further comprising a valve relief component including a valve relief hub and a valve relief shaft, wherein the valve relief component is configured to be disposed within the introducer hub such as to protect a distal portion of the delivery catheter from a seal of the introducer hub.
Clause 11. The system of clause 10, wherein the valve relief hub is configured to mate with the introducer hub, and the valve relief shaft is configured to extend through the introducer hub but not extend through the introducer shaft.
Clause 12. The system of clause 10 or 11, wherein the advancer hub is configured to be disposed proximal of the valve relief hub and the advancer shaft is configured to extend through the valve relief hub, the valve relief shaft, and the introducer shaft.
Clause 13. The system of clause 9 or any of clauses 9 to 12, wherein the delivery catheter includes a heart valve prosthesis mounted on a distal portion thereof, wherein a distal end the advancer shaft terminates proximal of the heart valve prosthesis.
Clause 14. The system of clause 13, wherein the delivery catheter is a balloon catheter and the heart valve prosthesis is mounted on a balloon of the balloon catheter.
Clause 15. A method of delivering a medical device to within a vasculature of a patient comprising:
   introducing an introducer into a vessel of the vasculature, the introducer including an introducer hub having a proximal portion outside of the vessel of the patient and an introducer shaft disposed within the vessel;
   advancing a delivery catheter through the introducer hub and the introducer shaft, wherein the delivery catheter includes an advancer mounted over an outer shaft of the delivery catheter, wherein the advancer includes an advancer hub and an advancer shaft extending distally from the advancer hub, wherein as the delivery catheter is advanced through the introducer hub and the introducer shaft the advancer is advanced such that the advancer shaft is extends through the introducer hub and the introducer shaft, and a proximal portion of the advancer hub is disposed proximal of the introducer hub; and
   peeling the advancer from the delivery catheter to remove the advancer from around the delivery catheter and from within the introducer when a distal portion of the delivery catheter has been advanced distal of a distal end of the introducer shaft.
Clause 16. The method of clause 15, further comprising inserting a valve relief component into the introducer, wherein the valve relief component includes a valve relief hub and a valve relief shaft extending distally from the valve relief hub,
   wherein the advancing the delivery catheter with the advancer mounted thereon comprises advancing the delivery catheter with the advancer mounted thereon through the valve relief component.
Clause 17. The method of clause 15, wherein the advancer shaft includes a split line and the advancer hub includes a parting line, wherein peeling the advancer comprises pulling the advancer hub apart such that the advancer hub and the advancer shaft split along the parting line and the split line.
Clause 18. The method of clause 17, wherein the advancer hub includes a hub body and two arms, wherein the parting line extends longitudinally along the hub body and the two arms extend radially outward from the hub body, and wherein peeling the advancer comprises pulling the two arms apart from each other to split the parting line and the split line.
Clause 19. The method of clause 15, wherein the advancer further comprises a seal configured to seal the advancer hub against the outer shaft of the delivery catheter, the seal including a seal parting line, wherein peeling the advancer comprises splitting of the seal along the seal parting line.
Clause 20. The method of clause 15, wherein the advancer further comprises a cap configured to couple the seal to the advancer hub, wherein the cap comprises two cap bodies configured to separate upon splitting of the advancer hub.

Further disclosed herein is an advancer including an advancer hub and an advancer shaft extending distally from the advancer hub. The advancer shaft includes a proximal end, a distal end, and a lumen extending from the proximal end to the distal end. The advancer shaft is configured to be disposed around an outer shaft of a delivery catheter, and includes a split line configured to enable splitting the shaft to enable removal of the shaft from the outer shaft. The advancer hub configured to be mounted on the outer shaft of the delivery catheter, and includes a parting line to enable splitting of the advancer hub.

## Claims

1. An advancer comprising:
a shaft including a proximal end, a distal end, and a lumen extending from the proximal end to the distal end, the shaft configured to be disposed around an outer shaft of a delivery catheter, the shaft including a split line configured to enable splitting the shaft to enable removal of the shaft from the outer shaft; and
a hub coupled to the proximal end of the shaft, the hub configured to be mounted on the outer shaft of the delivery catheter, the hub including a parting line to enable splitting of the hub.

2. The advancer of claim 1, further comprising a seal disposed within the hub, the seal configured to seal the hub against the outer shaft of the delivery catheter, the seal including a seal parting line to enable splitting of the seal.

3. The advancer of claim 2, wherein the seal includes two seal parting lines disposed about 180 degrees apart around a circumference of the shaft.

4. The advancer of claim 2 or 3, further comprising a cap configured to couple the seal to the hub, wherein the cap comprises two cap bodies configured to separate upon splitting of the hub.

5. The advancer of any preceding claim, wherein the shaft includes two split lines disposed about 180 degrees apart around a circumference of the shaft.

6. The advancer of any preceding claim, wherein the hub includes two parting lines disposed about 180 degrees apart around a circumference of the hub.

7. The advancer of any preceding claim, wherein the hub includes a hub body and two arms disposed about 180 degrees apart around a circumference of the hub body and extending radially outward form the hub body, wherein the parting line of the hub extend longitudinally along the hub body.

8. The advancer of any preceding claim, wherein the shaft includes a soft tip.

9. A system comprising:
an introducer, the introducer comprising an introducer hub and an introducer shaft extending distally from the introducer hub;
a delivery catheter, the delivery catheter including a handle and an outer shaft extending distally from the handle; and
an advancer, the advancer including an advancer hub and an advancer shaft extending distally therefrom,
wherein:
the advancer is mounted over the delivery catheter such that a distal portion of the outer shaft of the delivery catheter extends through the advancer hub and the advancer shaft;
the delivery catheter with the advanced mounted thereon is configured to be advanced through the introducer hub and the introducer shaft such that the advancer provides axial stiffness to the delivery catheter as the delivery catheter is advanced through the introducer hub and the introducer catheter; and
the advancer shaft and the advancer hub are peelable such that the advancer is configured to be removed from around the delivery catheter and from within the introducer after a distal portion of the delivery catheter is advanced distally past a distal end of the introducer shaft.

10. The system of claim 9, further comprising a valve relief component including a valve relief hub and a valve relief shaft, wherein the valve relief component is configured to be disposed within the introducer hub such as to protect a distal portion of the delivery catheter from a seal of the introducer hub.

11. The system of claim 10, wherein the valve relief hub is configured to mate with the introducer hub, and the valve relief shaft is configured to extend through the introducer hub but not extend through the introducer shaft.

12. The system of claim 10 or 11, wherein the advancer hub is configured to be disposed proximal of the valve relief hub and the advancer shaft is configured to extend through the valve relief hub, the valve relief shaft, and the introducer shaft.

13. The system of any one of claims 9 to 12, wherein the delivery catheter includes a heart valve prosthesis mounted on a distal portion thereof, wherein a distal end the advancer shaft terminates proximal of the heart valve prosthesis.

14. The system of claim 13, wherein the delivery catheter is a balloon catheter and the heart valve prosthesis is mounted on a balloon of the balloon catheter.
